(19) Europäisches Patentamt
European Patent Office
Office européen des brevets

(11) Numéro de publication : **0 336 823 B1**

# (12) FASCICULE DE BREVET EUROPEEN

(45) Date de publication du fascicule du brevet : **11.11.92 Bulletin 92/46**

(51) Int. Cl.⁵ : **C07C 11/02**

(21) Numéro de dépôt : **89400891.1**

(22) Date de dépôt : **30.03.89**

## (54) Procédé de production d'olefines à partir du gaz naturel.

(30) Priorité : **05.04.88 FR 8804588**

(43) Date de publication de la demande : **11.10.89 Bulletin 89/41**

(45) Mention de la délivrance du brevet : **11.11.92 Bulletin 92/46**

(84) Etats contractants désignés : **BE DE GB IT NL SE**

(56) Documents cités : **Eléments de la technique relevés: néant**

(73) Titulaire : **INSTITUT FRANCAIS DU PETROLE**
**4, Avenue de Bois Préau**
**F-92502 Rueil-Malmaison (FR)**

(72) Inventeur : **Cameron, Charles**
**28, rue Henri Barbusse**
**F-75005 Paris (FR)**
Inventeur : **Mimoun, Hubert**
**34, rue Hippolyte Bisson**
**F-92500 Rueil Malmaison (FR)**
Inventeur : **Robine, Alain**
**10, rue Massena**
**F-92500 Rueil Malmaison (FR)**
Inventeur : **Bonnaudet, Serge**
**4, rue de Cheroy**
**F-75017 Paris (FR)**
Inventeur : **Chaumette, Patrick**
**34, Côte de la Jonchère**
**F-78380 Bougival (FR)**
Inventeur : **Dang Vu, Quang**
**48 bis, Boulevard du Général Leclerc**
**F-92200 Neuilly (FR)**

**EP 0 336 823 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de production d'oléfines à partir du gaz naturel.

Le gaz naturel est une matière première fossile abondante constituée essentiellement de méthane, dont les réserves prouvées actuellement, de l'ordre de $10^{14}$ $m^3$ représentent environ 50 années de consommation mondiale. Les gisements de gaz contiennent souvent des quantités importantes d'éthane, de propane, d'autres alcanes supérieurs ainsi que d'autres constituants tels que $H_2O$, $CO_2$, $N_2$, $H_2S$ et He. La majorité du propane et autres alcanes supérieurs associés au gaz naturel sont liquifiés et désignés sous le nom de GPL (gaz de pétrole liquéfié). Dans les gisements riches en hélium (généralement plus de 0,3 % par volume), l'hélium est séparé à cause de sa haute valeur commerciale. On sépare également l'hydrogène sulfuré à cause de son caractère corrosif, et l'eau à cause de la formation d'hydrates qui nuisent au transport du gaz naturel. Le gaz naturel obtenu est alors qualifié de gaz non condensé, et contient principalement (par exemple 55-99 % en volume) du méthane ainsi que 1 à 25 % en volume d'éthane et éventuellement de faibles quantités de propane, d'azote et/ou de dioxyde de carbone.

La plus grande partie du gaz naturel est utilisée pour le chauffage individuel et industriel; cependant, certains procédés pour la tranformation du gaz naturel en hydrocarbures supérieurs existent.

La transformation directe du gaz naturel en éthylène serait un objectif hautement désirable, l'éthylène pouvant servir de matière première pour de nombreuses synthèses de produits importants.

La pyrolyse et la pyrolyse catalytique du méthane sont toutes deux des procédés permettant d'atteindre ce but. Cependant il s'agit de procédés très endothermiques qui exigent des quantités très importantes d'énergie. De plus, ces deux procédés produisent de grandes quantités non désirées de coke.

La pyrolyse de l'éthane est aussi un procédé connu qui est très endothermique et donc gros consommateur d'énergie. Cependant, comme la pyrolyse de l'éthane s'effectue à des températures plus faibles que celle du méthane, il n'est pas possible de convertir simultanément ces deux composés. Ainsi quand on opère à la température de conversion de l'éthane, le méthane présent dans la charge contenant l'éthane sort du réacteur essentiellement inchangé.

Pour produire de l'éthylène et d'autres hydrocarbures, on a aussi proposé le couplage oxydant du méthane, soit en mode séquentiel, soit en mode simultané.

La réaction du couplage oxydant en mode séquentiel consiste en l'oxydation du méthane par un agent réductible suivie de la réoxydation de cet agent, séparément, par l'oxygène de l'air. On a mentionné dans plusieurs brevets américains (voir, par exemple, 4 499 323, 4 523 049, 4 547 611, 4 567 307) l'utilisation de nombreux oxydes de métaux, notamment Mn, Ce, Pr, Sn, In, Ge, Pb, Sb, Bi, Tb, comme agents réductibles pour cette réaction.

La réaction du couplage oxydant en mode simultané (balayage d'un mélange de méthane et d'oxygène sur une masse de contact, C) peut s'écrire qualitativement :

$$CH_4 + O_2 \xrightarrow{C} C_2H_6 + C_2H_4 + \underset{\text{hydrocarbures}}{\text{autres}} + CO + CO_2 + H_2 + H_2O$$

Il a été mentionné dans plusieurs brevets (voir, par exemple, brevets européens EP 210 383 A2, EP 189 079 A1, EP 206 044 A1 et brevet mondial WO 86 07351) l'utilisation d'oxydes de terres rares, d'oxydes alcalins et alcalino-terreux, et d'oxydes de titane, zirconium, hafnium et zinc soit seuls, soit mélangés, comme catalyseurs pour la réaction du couplage oxydant du méthane en mode simultané.

Il faut remarquer que les travaux précédents concernant le couplage oxydant du méthane ont conduit à la formation de faibles rapports éthylène sur éthane, généralement compris entre environ 0,8 et 1,2 pour des sélectivités des produits $C_2$+ supérieures à environ 65 %. Des rapports éthylène sur éthane si faibles nécessitent la séparation de l'éthane de l'éthylène et la pyrolyse coûteuse de l'éthane en éthylène. Notons aussi que les travaux précédents sur le couplage oxydant du méthane n'ont pas pris en considération les autres constituants majeurs du gaz naturel tels que l'éthane, le propane et d'autres hydrocarbures saturés. Les procédés de couplage oxydant du méthane décrits ci-dessus sont peu appropriés à la conversion sélective du gaz naturel en oléfines à cause de la présence de quantités significatives d'hydrocarbures légers (tels que l'éthane et le propane) dans le gaz naturel. Les vitesses relatives d'oxydation des alcanes légers dans les conditions de couplage oxydant du méthane sont environ 15 à 100 fois plus grandes que celles de l'oxydation du méthane, c'est à dire que les hydrocarbures légers sont transformés en éthylène et en oxydes de carbone avant que le méthane ne commence à réagir. Ainsi, les procédés précédents ne peuvent être appliqués efficacement au gaz naturel.

Un des objectifs de la présente invention est de fournir une méthode de production d'oléfines, notamment d'éthylène, avec un rendement élevé, à partir du gaz naturel.

Un objectif supplémentaire de la présente invention est de fournir une méthode pour la déshydrogénation des alcanes qui sortent du réacteur d'oxydation du méthane sans la nécessité d'avoir un réacteur de pyrolyse indépendant.

Ces objectifs sont atteints par le présent procédé d'oxypyrolyse du gaz naturel qui comprend les étapes suivantes :

(1) la séparation du gaz naturel en deux fractions : une première fraction de gaz enrichie en méthane (qui de préférence est substantiellement exempte d'éthane, de propane et d'autres hydrocarbures associés au gaz naturel) et une deuxième fraction de gaz enrichie en éthane (qui peut ou non contenir d'autres hydrocarbures légers et/ou des gaz non hydrocarbonés) ;

(2) l'oxydation sélective de la première fraction de gaz contenant du méthane par l'oxygène moléculaire, en présence d'une masse de contact, pour former un effluent contenant comme produits principaux des hydrocarbures $C_2$ et de l'eau ;

(3) l'addition de la deuxième fraction de gaz, au moins, à l'effluent de la réaction d'oxydation sélective du méthane à un endroit où au moins environ 80 % en volume de l'oxygène moléculaire introduit à l'étape (2) ont été consommés ; et (4) la pyrolyse contrôlée du mélange du dit effluent et de la dite deuxième fraction de gaz, qui produit un mélange gazeux riche en oléfines et hydrogène.

Le procédé d'oxypyrolyse du gaz naturel selon l'invention présente les principaux avantages suivants, par rapport aux procédés existants impliquant le couplage oxydant du méthane ou la pyrolyse et pyrolyse catalytique du méthane et de l'éthane :

a) la chaleur dégagée pendant la réaction d'oxydation (approximativement 250 kJ par mole de méthane ayant réagi) est immédiatement utilisée pour la pyrolyse des alcanes de l'effluent et des autres alcanes ajoutés (tels que l'éthane, le propane, le butane et le naptha).

b) les rapports oléfines (notamment l'éthylène et le propylène) sur alcanes $C_2$+ (notamment l'éthane et le propane) sont presque toujours supérieurs à 1,5.

c) des rapports appropriés oléfines sur alcanes peuvent être obtenus en contrôlant le temps de résidence dans l'étape de pyrolyse (4) et la température de l'effluent gazeux qui résulte immédiatement de l'oxydation sélective du méthane.

d) l'effluent de la réaction d'oxydation peut servir comme diluant de déshydrogénation pour une réaction de pyrolyse de l'éthane et d'autres hydrocarbures saturés qui peuvent être introduits après qu'ait eu lieu la réaction initiale d'oxydation.

e) la nécessité de convertir séparément l'éthane en éthylène par un procédé de pyrolyse conventionnelle est éliminée.

f) le procédé permet de traiter la totalité du gaz naturel et non pas seulement le méthane contenu dans le gaz naturel.

Trois éléments importants doivent être considérés pour que le procédé fonctionne de façon optimale ; ce sont : 1) la masse de contact, 2) la deuxième fraction de gaz qui contient des hydrocarbures saturés, et 3) le contrôle de la température et du temps de résidence, du mélange de l'effluent et du gaz ajouté entre l'endroit où le mélange est réalisé et la trempe thermique effectuée à la sortie du réacteur.

En ce qui concerne la masse de contact pour la réaction d'oxydation sélective du méthane en hydrocarbures supérieurs, bien que l'on puisse utiliser toute masse de contact connue pour cet usage, on préfère une masse de contact qui :

(a) soit capable d'opérer dans les conditions normales du couplage oxydant, c'est-à-dire à des températures comprises entre environ 700 et 950 °C,

(b) produise des produits en $C_2$+ avec une sélectivité d'au moins environ 65 % pour une conversion de 15 % environ du méthane, et

(c) maintienne son activité et sa sélectivité pendant de nombreuses heures d'opération.

Les masses de contact qui correspondent aux conditions ci-dessus et dont l'emploi est donc préféré sont celles qui, en général, contiennent des oxydes et/ou des carbonates de métaux alcalins (tels que le lithium, le sodium, le potassium, le rubidium et le césium), de métaux alcalino-terreux (tels que le béryllium, le magnésium, le calcium, le strontium et le baryum), et de métaux des terres rares (tels que l'yttrium, le lanthane, le praséodyme, le néodyme, le samarium, le gadolinium, le terbium, le dysprosium, le holmium, l'erbium, le thulium, l'ytterbium, l'europium et le lutétium) soit seuls (comme dans les cas des terres rares et des alcalino-terreux) soit en mélange (comme dans les cas des métaux alcalino-terreux dopés avec des métaux alcalins et des métaux des terres rares dopés avec des métaux alcalins et/ou alcalino-terreux). D'autres masses de contact qui correspondent aux conditions ci-dessus sont celles qui contiennent des oxydes et/ou des carbonates de titane, zirconium, hafnium, manganèse, cérium, étain, indium, germanium, plomb, antimoine, zinc et

bismuth, préférablement avec un ou plusieurs oxydes et/ou carbonates de métaux alcalins, alcalino-terreux, terres rares et silice. Les masses de contact sus-désignées sont efficaces seules ou dopées avec des halogénures.

Il est aussi intéressant que les masses de contact pour l'oxydation sélective du méthane :

(a) soient effectives pendant des temps de contact très courts, généralement moins de 300 millisecondes environ,

(b) soient capables d'effectuer la consommation d'au moins environ 80 % (de préférence au moins environ 95 %) en volume de l'oxygène moléculaire dans la charge pendant des temps de contact très courts.

Les masses de contact qui sont particulièrement intéressantes sont les suivantes :

**(a)** Les masses de contact contenant des composés alcalino-terreux et de terres rares et correspondant approximativement à la formule suivante :

$$T_aLn_2O_{b-c}(CO_3)_c$$

où T représente un ou plusieurs métaux alcalino-terreux tels que le béryllium, le magnésium, le calcium, le strontium et le baryum, Ln représente un ou plusieurs métaux de terres rares tels que l'yttrium, le lanthane, le praséodyme, le néodyme, le samarium, l'europium, le gadolinium, le terbium, le dysprosium, le holmium, l'erbium, le thulium, l'ytterbium et le lutetium, a = 0,001 à 1000, b = 3 + a, c = 0,1 à b. Les métaux de terres rares préférables sont le lanthane, le néodyme et le samarium, seuls ou en mélange. Le métal de terre rare préféré est le lanthane.

**(b)** Les masses de contact contenant des composés alcalino-terreux, de terres rares et du groupe IVA et correspondant approximativement à la formule suivante :

$$T_aM_dLn_2O_{e-f}(CO_3)_f$$

où T représente un ou plusieurs métaux alcalino-terreux, M représente un ou plusieurs métaux du groupe IVA tels que le titane, le zirconium et le hafnium, Ln représente un ou plusieurs métaux de terres rares, a = 0,001 à 1000, d = 0,001 à 2, e = 3 + a +2d - z, f = 0,01 à e, z = 0 à 0,5d. Dans cette formule, z représente une valeur de 0 à 0,5d, donc z est égal à 0 quand l'état d'oxydation de tous les métaux du groupe IVA est de +4, 0,5d quand l'état d'oxydation de ces métaux est de +3, et entre 0 et 0,5d quand un mélange des états d'oxydation +3 et +4 de ces métaux est présent. Les métaux de terres rares préférés sont le lanthane, le néodyme et le samarium, et plus particulièrement le lanthane.

Les masses de contact **(a)** et **(b)** ne sont toutefois pas limitées aux formules données ci-dessus. En effet l'effet bénéfique de la présence de métaux de terre rares et de métaux alcalino-terreux et éventuellement de métaux du groupe IV A se manifeste quelle que soit la forme sous laquelle ces métaux sont présents, bien que l'on préfère la forme oxyde et/ou carbonate.

**(c)** Les masses de contact contenant deux ou plusieurs métaux alcalino-terreux et répondant approximativement à la formule suivante :

$$T_gT'_{2-g}O_h(CO_3)_{2-h}$$

où T représente un ou plusieurs métaux alcalino-terreux qui sont différents de T′, tels que le béryllium, le magnésium, le calcium, le strontium et le baryum, T′ représente un métal alcalino-terreux qui est un des métaux alcalino-terreux connu pour former des carbonates stables à haute température tels que le calcium, le strontium et le baryum, g = 0,1 à 1,8, h = 0 à 2. Les métaux alcalino-terreux T′ préférables sont le strontium et le baryum.

Les masses de contact **(c)** ne sont toutefois pas limitées à la formule donnée ci-dessus. En effet l'effet bénéfique de la présence d'au moins deux métaux alcalino-terreux (où au moins un composant tel que le calcium, le strontium ou le baryum) se manifeste quelle que soit la forme sous laquelle ces métaux sont présents, bien que l'on préfère la forme carbonate ou oxyde et carbonate.

En ce qui concerne le gaz ajouté (qui est riche en éthane, provient au moins en partie du gaz naturel, et peut contenir d'autres hydrocarbures légers, du naptha et/ou des gaz non hydrocarbonés), on a constaté que l'addition de celui-ci au gaz contenant du méthane entraine une conversion faible du méthane et une grande conversion des hydrocarbures ajoutés quand l'ensemble de ces gaz est mis en contact avec un gaz contenant de l'oxygène et une masse de contact. A cause de la réactivité plus élevée de l'éthane (et des autres hydrocarbures dans le gaz ajouté) que celle du méthane, l'éthane est oxydé en éthylène, CO et $CO_2$ avant que le méthane n'ait suffisamment réagi.

Cette façon insatisfaisante de procéder est donc exclue de la présente invention, selon laquelle tout au contraire, on introduit le dit gaz ajouté dans le réacteur d'oxypyrolyse après le lit de la masse de contact ou en un endroit de celle-ci où au moins environ 80 % (de préférence au moins environ 95 %) de l'oxygène moléculaire ont déjà été consommés lors de l'oxydation sélective du méthane. Une certaine fraction de l'éthane et des autres hydrocarbures saturés de l'effluent d'oxydation sélective du méthane et une certaine fraction du gaz contenant des hydrocarbures ajoutés peuvent être déshydrogénées par une réaction de pyrolyse pour former un

gaz riche en oléfines en utilisant la chaleur générée par la réaction d'oxydation du méthane et en contrôlant le temps de résidence du mélange gazeux présent après avoir mélangé l'effluent avec le dit gaz ajouté.

Le mélange de gaz soumis à l'oxydation (étape 2 du procédé) peut être utilisé sans diluant ou dilué par des gaz inertes tels que l'azote, le dioxyde de carbone ou la vapeur d'eau. Pour des raisons de sécurité, la teneur en oxygène dans le méthane ne peut dépasser 40 % molaire ; elle peut donc être comprise entre environ 0,1 et 40 % molaire, de préférence entre 5 et 25 % molaire.

La température de la réaction d'oxypyrolyse (étapes 2 et 4) est généralement comprise entre 750 °C et 1200 °C, de préférence entre 750 °C et 950 °C.

La pression totale (étapes 2 et 4) peut être par exemple de 1 à 100 bars, et notamment de 1 à 20 bars. Le temps de contact de couplage oxydant du méthane (c'est-à-dire le temps nécessaire pour effectuer la consommation d'au moins environ 80 % de l'oxygène moléculaire dans la charge) est compris entre $10^{-6}$ sec. et 1 sec., de préférence entre $10^{-6}$ et $10^{-1}$ sec. Le temps de résidence de la réaction de pyrolyse est compris entre environ $10^{-3}$ sec. et 10 sec., et notamment entre environ $10^{-2}$ et 2 sec.

En ce qui concerne le contrôle de la température et du temps de résidence du dit mélange gazeux entre l'endroit où le mélange est effectué et la trempe thermique, plusieurs types de réacteurs peuvent être utilisés afin d'obtenir le rapport oléfines sur alcanes désiré.

Les exemples suivants non limitatifs illustrent les différents modes de réalisation qui peuvent être employés pour atteindre des rapports bien définis oléfines sur alcanes à partir du gaz naturel.

Une mise en oeuvre préférée de la présente invention consiste à utiliser un réacteur à lit fixe. Dans cette mise en oeuvre, le gaz riche en méthane est préchauffé à une température qui n'excède généralement pas 600 °C environ, puis mélangé avec l'oxygène avant d'être mis en contact sur le lit fixe. La masse de contact (qui peut être présente sous forme de poudre, de granulés, d'extrudés, de pastilles, de monolithe ou supportée sur des oxydes et/ou carbonates de métaux alcalino-terreux et/ou de métaux des terres rares, sur zircone, silice, alumine, quartz ou sur des monolithes tels que par exemple la cordiérite, la mullite ou l'alumine alpha) est chauffée, par l'exothermicité de la réaction d'oxydation, à une température d'environ 750 à 1200 °C. La température de la masse de contact est une fonction de la température initiale du gaz contenant le mélange de méthane et d'oxygène moléculaire ; de la quantité et de l'enthalpie de l'effluent et du mélange contenant le méthane et l'oxygène ; et de la capacité calorifique de l'effluent.

Le dit gaz ajouté est alors introduit dans le réacteur après le lit de contact ou en un endroit où au moins 80 % (de préférence au moins 95 %) de l'oxygène moléculaire ont été consommés. Le dit gaz ajouté peut être introduit à température ambiante ou préchauffé à une température inférieure à la température de l'effluent. Dans les deux cas, le dit gaz ajouté sert à abaisser la température de l'effluent, et produit alors un mélange qui est à une température inférieure à la température de l'effluent. La température du mélange peut être contrôlée en ajustant les températures de la charge du méthane et du dit gaz ajouté et le pourcentage initial d'oxygène moléculaire. Le temps de résidence du mélange (de l'effluent et du dit gaz ajouté) est fixé en fonction de la température du mélange et de la sévérité de la pyrolyse désirée. La réaction de pyrolyse peut être effectuée soit en absence de masse de contact soit en présence d'une masse de contact (en lit fixe de préférence) telle que l'une de celles antérieurement décrites pour l'oxydation sélective du méthane.

Une autre mise en oeuvre préférée de la présente invention utilise un réacteur à lit bouillonnant. Dans ce cas, les conditions opératoires sont les mêmes sauf que la masse de contact (qui peut être présente sous forme de poudre ou de granules) reste en suspension.

Une autre mise en oeuvre préférée de la présente invention utilise un réacteur à lit circulant ou à lit fluidisé. Dans cette mise en oeuvre le dit gaz du méthane est préchauffé à une température qui n'excède pas généralement 600 °C environ, puis mélangé avec le gaz contenant l'oxygène moléculaire avant d'être introduit dans le fond du réacteur. La charge du méthane et de l'oxygène aspire la masse de contact chaude qui est entrainée jusqu' au sommet du réacteur. La masse de contact (qui peut être présente sous forme de poudre ou de granules ou supportée sur des oxydes et/ou carbonates de métaux alcalino-terreux et des terres rares, sur zircone, silice, alumine, quartz, alumine alpha ou sur d'autres supports) soit descend au fond du réacteur par un chemin différent où elle est aspirée de nouveau soit est transférée à l'extérieur du réacteur pour un traitement ultérieur avant d'être aspirée de nouveau.

Le dit gaz ajouté est alors introduit dans le réacteur à un endroit où au moins 80 % de l'oxygène moléculaire ont été consommés. Le dit gaz ajouté peut être introduit à température ambiante ou préchauffé à une température inférieure à la température de l'effluent. Comme ci-dessus, le temps de résidence du mélange (de l'effluent et du dit gaz ajouté) est fixé en fonction de la température du mélange et de la sévérité de la pyrolyse désirée.

Les mises en oeuvres ne sont toutefois pas limitées aux exemples décrits ci-dessus. En effet, les effets bénéfiques de l'addition d'un gaz contenant au moins un hydrocarbure saturé en un endroit où au moins environ 80 % (de préférence au moins 95 %) de l'oxygène moléculaire ont été consommés (par la réaction d'oxydation

sélective du méthane), se manifestent quelle que soit la forme du réacteur, bien que l'on préfère les formes de lit fixe, bouillonnant et circulant.

Quel que soit le type de réacteur utilisé, on peut employer des sections réactionnelles distinctes pour les étapes (2) et (4) du procédé (figure 1) ou n'utiliser qu'une seule section comportant deux zones successives de réaction (figure 2).

**EXEMPLES** Les exemples sont illustrés par les figures 1 et 2.

Un courant de gaz naturel (2) est fractionné dans le séparateur (1) en méthane (3) et hydrocarbures supérieurs (4). Le courant de méthane (3) alimente un tube vertical en quartz de 1300 mm de hauteur et 13 mm de diamètre intérieur. Le réacteur reçoit aussi de l'oxygène par la ligne (6). Dans la partie inférieure du tube (5) on dispose successivement, du bas vers le haut :

- des grains de quartz (granulométrie = 1 à 2 mm) jusqu'à 465 mm de haut,
- un tampon de laine de quartz (5 mm d'épaisseur),
- le lit de contact (30 mm d'épaisseur),
- un second tampon de laine de quartz,
- d'autres grains de quartz pour atteindre 550 mm de haut.

On ajoute alors le courant (7) d'éthane et hydrocarbures supérieurs et le mélange résultant poursuit son cheminement dans la partie supérieure du tube (8) (entre les niveaux 550 mm et 1300 mm).

Les deux sections successives sont disposées chacune dans un four et la partie de canalisation entre les deux fours, d'une longueur de 40 mm, est isolée thermiquement.

Le gaz effluent est trempé (9) et évacué (10) si l'on désire l'utiliser tel quel. On peut également le recycler (11) ; il est alors souvent avantageux de soumettre le gaz à divers fractionnements tels que :

- purge, permettant d'éviter l'accumulation de gaz tels que $H_2$, He, $N_2$ ou CO ;
- lavage aux amines, permettant d'éliminer $CO_2$ ;
- méthanation permettant de convertir CO et $H_2$ en $CH_4$ et $H_2O$.

Ces fractionnements sont globalement représentés par l'unité (12), et le gaz séparé est éliminé par la ligne (13). Le gaz restant, renfermant des hydrocarbures non-convertis et de l'éthylène, est envoyé au séparateur (1) par la ligne (14).

Les hydrocarbures ($C_2$+) provenant du séparateur (1) sont soumis à un fractionnement dans l'unité (15), de façon à retirer au moins la majeure partie de l'éthylène et à envoyer dans la conduite (7) un courant riche en hydrocarbures saturés. L'unité (15) peut être toute unité capable de séparer des oléfines. Ce peut être une absorption dans l'acide sulfurique, une distillation, une oligomérisation des oléfines en carburants liquides, par exemple en essence et/ou gas oil. Ces produits sont déchargés par la ligne (16).

La ligne 17 permet de prélever des échantillons, notamment pour mesurer la conversion de l'oxygène. Il convient de signaler ici une variante du schéma ci-dessus qui a donné des résultats comparables (figure 2). Les deux sections 5 et 8 du tube de réaction sont disposées directement à la suite l'une de l'autre, l'ensemble étant dans un four unique.

Dans la suite on appellera premier réacteur et second réacteur respectivement les sections inférieure oxydante (5) et supérieure pyrolytique (8) du tube de quartz ci-dessus.

Dans tous les exemples le lit de contact a un volume de 3,2 ml. Les lits de contact sont préparés de la façon suivante :

Masse de contact A)

La masse de contact est préparée en calcinant à 600 °C à l'air pendant deux heures, un mélange mécanique de $SrCO_3$ et de $La_2(CO_3)_3$ dans un rapport atomique Sr/La de 0,5. Le lit de contact est constitué de 0,3 g de masse de contact diluée dans 3,2 ml de grains d'alumine tabulaire (dont la taille est comprise entre 0,3 et 0,6 mm).

Masse de contact B)

Des grains de magnésie (dont la taille est comprise entre 0,5 et 1 mm) sont calcinés à 600 °C à l'air pendant deux heures puis laissés à refroidir dans un dessicateur à température ambiante. Après quoi, pendant une minute à 40 °C et sous agitation, ils sont imprégnés d'une solution aqueuse de $Ba(NO_3)_2$ et de $La(NO_3)_3$, dans un rapport atomique Ba/La = 0,5. Ensuite, la masse de contact supportée est calcinée à 800 °C pendant deux heures. Le lit de contact contient uniquement la masse de contact supportée sans diluant.

Masse de contact C)

Le lit de contact est préparé comme décrit dans B, mais en utilisant une solution aqueuse de $Sr(NO_3)_2$ et de $La(NO_3)_3$ dans un rapport atomique Sr/La = 0,5.

Les débits rapportés ne tiennent pas compte des autres hydrocarbures supérieurs, notamment les $C_4$ et les $C_6$, qui ont été détectés à des concentrations significatives par analyse gazeuse de l'effluent.

**EXEMPLES 1 - 4**

On opère avec un gaz naturel ayant un rapport volumique $C_2H_6/(C_2H_6 + CH_4)$ = 9,1 % et en utilisant le lit de contact A.

Le temps de résidence dans le lit de contact de la section inférieure oxydante (5) (dans les exemples 1, 2 et 4) est de l'ordre de 0,044 sec. Cette valeur est basée sur le volume (3,2 ml) et la température (880 °C) du lit de contact et le débit de gaz au point 17 de l'exemple 1 (2,75 g mol/h).

Dans l'exemple 3, ce gaz est envoyé en totalité, sans fractionnement, à travers les deux réacteurs (5 puis 8).

Dans les autres cas (exemples 1, 2 et 4), le gaz est d'abord fractionné en méthane et éthane. Dans l'exemple 1, le méthane est envoyé au réacteur (5), le réacteur (8) n'étant pas utilisé. Dans l'exemple 2, le méthane traverse successivement les deux réacteurs (5 puis 8); l'éthane n'est utilisé ni dans l'exemple 1 ni dans l'exemple 2.

Dans l'exemple 4, selon l'invention, le méthane traverse successivement les réacteurs (5 et 8) et l'éthane est introduit à l'entrée du second réacteur (8), en un point où la conversion de l'oxygène est de 99,6 %. Dans tous les cas, on utilise, dans le réacteur (5), le lit de contact A avec un rapport $O_2/(O_2 + CH_4)$ de 9 % en volume à l'entrée du réacteur (5).

Le temps de résidence dans la section supérieure pyrolytique (8) de cet exemple est de l'ordre de 0,79 sec. Le temps de résidence est calculé en utilisant le volume de la section 8 dans le four (diamètre = 13 mm, hauteur = 500 mm, volume = 66,4 $cm^3$) et le débit par seconde du gaz qui sort du réacteur (8) à une température de 850 °C (débit total = 3,28 mol/h, débit par seconde = 84 $cm^3$/sec).

Les conditions opératoires et les résultats après trempe (9) sont donnés dans le tableau 1.

**EXEMPLE 5 - 8**

Les exemples 5 - 8, tableau 2, sont respectivement effectués dans les mêmes conditions que les exemples 1 - 4 ; toutefois le rapport $O_2/(O_2 + CH_4)$ est de 13 %. Il est à noter que le mélange gazeux, issu de la réaction de pyrolyse est plus riche en produits $C_2$ + et en hydrogène, quand l'éthane est ajouté après que soit consommée la plus grande partie (99,7 %) de l'oxygène moléculaire (exemple 8).

**EXEMPLES 9 - 16**

Dans les exemples 9 - 16, tableau 3, les réactions sont effectuées en utilisant le lit de contact B. Dans ces exemples la température du lit (1er réacteur) est maintenue à 880 °C et le débit du méthane est fixé à 1000 ml/min. On montre que le rapport oléfines sur alcanes est indépendant du pourcentage de l'oxygène moléculaire dans la charge et de la quantité de l'éthane ajouté après le lit du contact, mais directement lié à la température du four de pyrolyse (réacteur 2). Le pourcentage de l'oxygène moléculaire consommé dans ces exemples est exactement le même au niveau du tube de prélèvement (entre les 2 réacteurs) et à la sortie du 2ème réacteur.

**EXEMPLES 17 - 22**

Les exemples 17-22 sont effectués en utilisant le lit de contact C en conservant la température du lit (1er réacteur) à 880 °C et celle du 2ème réacteur à 850 °C et en conservant le débit du méthane à 1000 ml/min et celui de l'oxygène moléculaire à 150 ml/min. Le tableau 4 montre que les débits d'oxygène moléculaire, d'oxyde de carbone et de dioxyde de carbone ne changent pas quand le débit d'éthane ajouté varie.

**EXEMPLE 23**

L'exemple 23 est effectué en utilisant les mêmes conditions précédemment décrites dans l'exemple 17. Une coupe de naptha (intervalle de température = 25° - 130 °C, pourcentage en paraffines = 81 %) est ajoutée

dans la partie (7) du réacteur avec un débit liquide de 0,2 ml/min, après que soit consommée la plus grande partie (99,8 %) de l'oxygène moléculaire. L'analyse gazeuse de l'effluent montre que celui-ci est constitué de 0,179 mol/h d'éthylène, 0,037 mol/h de propylène, 0,058 mol/h d'éthane et 0,003 mol/h de propane. Le rapport oléfines sur alcanes pour les produits $C_2$ + $C_3$ est 3,5.

TABLEAU N° 1

| EXEMPLE | 1 | 2 | 3 | 4 |
|---|---|---|---|---|
| Température du lit (1er réacteur) °C | 881 | 880 | 880 | 880 |
| Température du 2ème réacteur | a | 850 | 850 | 850 |
| Débit (ml/min) $CH_4$ | 1000 | 1000 | 1000 | 1000 |
| Débit (ml/min) $O_2$ | 99 | 99 | 99 | 99 |
| Débit (ml/min)$C_2H_6$ à l'entrée du 1er réacteur | 0 | 0 | 100 | 0 |
| Débit (ml/min)$C_2H_6$ introduit entre les 2 réacteurs | 0 | 0 | 0 | 100 |
| Débits (mol/h) à la sortie | | | | |
| $H_2$ | 0,064 | 0,124 | 0,222 | 0,303 |
| $O_2$ | 0,001 | 0,001 | 0,002 | 0,001 |
| CO | 0,016 | 0,017 | 0,039 | 0,018 |
| $CO_2$ | 0,060 | 0,059 | 0,064 | 0,059 |
| $C_2H_4$ | 0,066 | 0,105 | 0,204 | 0,267 |
| $C_2H_6$ | 0,082 | 0,036 | 0,067 | 0,094 |
| $C_3H_6$ | 0,004 | 0,008 | 0,012 | 0,011 |
| $C_3H_8$ | 0,004 | 0 | 0 | 0 |
| $CH_4$ | 2,104 | 2,118 | 2,319 | 2,168 |
| $H_2O$ | 0,358 | 0,359 | 0,325 | 0,358 |
| Total $C_2$+($C_2$ + $C_3$) | 0,156 | 0,149 | 0,283 | 0,372 |
| % conversion $CH_4$ | 15,8 | 15,3 | c | 15,3 d |
| % conversion $O_2$ | 99,6 | 99,6 | 99,2 | 99,6 |
| % conversion $C_2H_6$ ajouté | b | b | c | 76,8 d |
| Rapport oléfines/alcanes $C_2$+ | 0,8 | 3,1 | 3,2 | 3,0 |

***** **a)** pas utilisé ; **b)** pas applicable dans ces cas ; **c)** les conversions d'éthane et de méthane ne peuvent pas être directement calculées ; **d)** la conversion du méthane est égale à celle de l'exemple 2 ; l'augmentation de débit de méthane en sortie des réacteurs, dans l'exemple 4 par rapport à l'exemple 2, est imputée à la conversion partielle de l'éthane en méthane.

TABLEAU N° 2

| EXEMPLE | 5 | 6 | 7 | 8 |
|---|---|---|---|---|
| Température du lit 1er réacteur °C | 880 | 880 | 880 | 880 |
| Température du 2ème réacteur | a | 850 | 850 | 850 |
| Débit (ml/min) $CH_4$ | 1000 | 1000 | 1000 | 1000 |
| Débit (ml/min) $O_2$ | 150 | 150 | 150 | 150 |
| Débit (ml/min)$C_2H_6$ à l'entrée du 1er réacteur | 0 | 0 | 100 | 0 |
| Débit (ml/min)$C_2H_6$ introduit entre les 2 réacteurs | 0 | 0 | 0 | 100 |
| Débits (mol/h) à la sortie | | | | |
| $H_2$ | 0,093 | 0,160 | 0,243 | 0,318 |
| $O_2$ | 0,001 | 0,001 | 0,004 | 0,001 |
| CO | 0,038 | 0,041 | 0,069 | 0,043 |
| $CO_2$ | 0,105 | 0,101 | 0,109 | 0,103 |
| $C_2H_4$ | 0,091 | 0,133 | 0,219 | 0,292 |
| $C_2H_6$ | 0,091 | 0,042 | 0,066 | 0,104 |
| $C_3H_6$ | 0,005 | 0,009 | 0,013 | 0,010 |
| $C_3H_8$ | 0,003 | 0 | 0 | 0 |
| $CH_4$ | 1,969 | 1,981 | 2,213 | 2,032 |
| $H_2O$ | 0,500 | 0,505 | 0,455 | 0,499 |
| Total $C_2$+($C_2$ + $C_3$) | 0,190 | 0,184 | 0,298 | 0,406 |
| % conversion $CH_4$ | 21,2 | 20,8 | c | 20,8 d |
| % conversion $O_2$ | 99,7 | 99,7 | | 99,7 |
| % conversion $C_2H_6$ ajouté | b | b | c | 75,2 d |
| Rapport oléfines/alcanes $C_2$+ | 1,0 | 3,4 | 3,5 | 2,9 |

* **a)** pas utilisé ; **b)** pas applicable dans ces cas ; **c)** les conversions d'éthane et de méthane ne peuvent pas être directement calculées ; **d)** la conversion du méthane est égale à celle de l'exemple 6 ; l'augmentation de débit de méthane en sortie des réacteurs, dans l'exemple 8 par rapport à l'exemple 6, est imputée à la conversion partielle de l'éthane en méthane.

TABLEAU N° 3

| EXEMPLE | 9 | 10 | 11 | 12 | 13 | 14 | 15 | 16 |
|---|---|---|---|---|---|---|---|---|
| Température du 2ème réacteur (°C) | 850 | 850 | 880 | 880 | 850 | 850 | 880 | 880 |
| Débit (ml/min) $O_2$ | 150 | 150 | 150 | 150 | 99 | 99 | 99 | 99 |
| Débit (ml/min) $C_2H_6$ introduit entre les deux réacteurs | 50 | 100 | 50 | 100 | 50 | 100 | 50 | 100 |
| Débit (mol/h) à la sortie | | | | | | | | |
| $H_2$ | 0,218 | 0,310 | 0,274 | 0,387 | 0,199 | 0,299 | 0,255 | 0,366 |
| $O_2$ | 0,001 | 0,001 | 0,001 | 0,001 | 0,001 | 0,001 | 0,001 | 0,001 |
| CO | 0,021 | 0,020 | 0,020 | 0,020 | 0,010 | 0,011 | 0,011 | 0,011 |
| $CO_2$ | 0,114 | 0,112 | 0,112 | 0,113 | 0,060 | 0,060 | 0,060 | 0,59 |
| $C_2H_4$ | 0,211 | 0,297 | 0,223 | 0,320 | 0,188 | 0,277 | 0,200 | 0,294 |
| $C_2H_6$ | 0,071 | 0,106 | 0,036 | 0,050 | 0,066 | 0,095 | 0,032 | 0,046 |
| $C_3H_6$ | 0,012 | 0,012 | 0,014 | 0,014 | 0,012 | 0,013 | 0,014 | 0,016 |
| % conversion $O_2$ (17) | 99,7 | 99,7 | 99,7 | 99,7 | 99,6 | 99,6 | 99,6 | 99,6 |
| Rapport oléfines/alcanes $C_2$+ | 3,1 | 2,9 | 6,6 | 6,7 | 3,0 | 3,1 | 6,7 | 6,7 |

## TABLEAU N° 4

| EXEMPLE | 17 | 18 | 19 | 20 | 21 | 22 |
|---|---|---|---|---|---|---|
| Débit (ml/min) $C_2H_6$ introduit entre les deux réacteurs | 0 | 11,1 | 23,3 | 47,9 | 71,4 | 93,4 |
| Débit (mol/h) à la sortie | | | | | | |
| $H_2$ | 0,118 | 0,136 | 0,156 | 0,202 | 0,247 | 0,287 |
| $O_2$ | 0,001 | 0,001 | 0,001 | 0,001 | 0,001 | 0,001 |
| CO | 0,022 | 0,022 | 0,022 | 0,022 | 0,022 | 0,022 |
| $CO_2$ | 0,112 | 0,112 | 0,112 | 0,112 | 0,112 | 0,112 |
| $C_2H_4$ | 0,122 | 0,139 | 0,157 | 0,200 | 0,244 | 0,280 |
| $C_2H_6$ | 0,039 | 0,048 | 0,058 | 0,073 | 0,091 | 0,104 |
| $C_3H_6$ | 0,008 | 0,010 | 0,011 | 0,011 | 0,012 | 0,012 |
| % conversion $O_2$ (17) | 99,7 | 99,7 | 99,7 | 99,7 | 99,7 | 99,7 |
| Rapport oléfines/alcanes $C_2^+$ | 3,3 | 3,1 | 2,9 | 2,9 | 2,8 | 2,8 |

## Revendications

1. Procédé de production d'oléfines à partir de gaz naturel renfermant du méthane et de l'éthane, caractérisé en ce que :
   a) on sépare le gaz naturel (2) en deux fractions, une première fraction de gaz enrichie en méthane (3) et une deuxième fraction de gaz enrichie en éthane (4),
   b) on oxyde (5) la première fraction de gaz par l'oxygène moléculaire (6), en présence d'une masse de contact permettant le couplage oxydant du méthane, en hydrocarbures supérieurs,
   c) on mélange l'effluent de l'étape (b) avec la fraction (7) de gaz enrichie en éthane de l'étape (a), quand au moins 80 % de l'oxygène moléculaire ont déjà été consommé dans l'étape (b), et
   d) on pyrolyse (8) le mélange résultant de l'étape (c).

2. Procédé selon la revendication 1 dans lequel on mélange dans l'étape (b) la fraction enrichie en méthane (3) de l'étape (a) avec de la vapeur d'eau.

3. Procédé selon l'une des revendications 1 et 2 dans lequel on mélange dans l'étape (c) la fraction enrichie en éthane (4) de l'étape (a) avec un ou plusieurs hydrocarbures saturés ne provenant pas du gaz naturel.

4. Procédé selon l'une des revendications 1 et 2 dans lequel on mélange dans l'étape (c) la fraction enrichie en éthane (4) de l'étape (a) avec un ou plusieurs gaz non hydrocarbonés.

5. Procédé selon l'une des revendications 1 à 4 dans lequel on effectue les étapes (b), (c) et (d) dans un réacteur à lit fluidisé circulant en présence de masse de contact.

6. Procédé selon l'une des revendications 1 à 4 dans lequel on effectue les étapes (b), (c) et (d) dans un réacteur à lit bouillonnant en présence de masse de contact.

7. Procédé selon l'une des revendications 1 à 4 dans lequel on effectue l'étape (b) dans un réacteur à lit fixe en présence de masse de contact.

8. Procédé selon l'une des revendications 1 à 4 dans lequel on effectue l'étape (b) et au moins l'une des étapes (c) et (d) dans un réacteur à lit fixe en présence de masse de contact.

9. Procédé selon l'une des revendications 7 et 8 dans lequel la masse de contact en lit fixe est supportée sur un monolithe.

10. Procédé selon l'une des revendications 7 et 8 dans lequel la masse de contact en lit fixe est sous forme de monolithe.

11. Procédé selon l'une des revendications 5 à 10 dans lequel la masse de contact correspond à la formule $T_aLn_2O_{b-c}(CO_3)_c$ , où T représente au moins un métal alcalino-terreux, Ln représente au moins un métal de terre rare, a = 0,001 à 1000, b = 3 + a, c= 0,1 à b.

12. Procédé selon l'une des revendications 5 à 10 dans lequel la masse de contact correspond à la formule $T_aM_dLn_2O_{e-f}(CO_3)_f$, où T représente au moins un métal alcalino-terreux, M représente au moins un métal du groupe IV A, Ln représente au moins un métal de terre rare, a = 0,001 à 1000, d = 0,001 à 2, e = 3 + a + 2d-z, f = 0,1 à e, z = 0 à 0,5d.

13. Procédé selon l'une des revendications 5 à 10 dans lequel la masse de contact résulte de l'imprégnation de magnésie par une solution aqueuse de nitrate de lanthane et de nitrate de baryum ou strontium.

14. Procédé selon l'une des revendications 1 à 13 dans lequel on fractionne l'effluent de l'étape (d), puis la fraction contenant CO est convertie en $CH_4$ et $H_2O$ par $H_2$ et enfin on renvoie l'effluent gazeux résultant vers l'étape (a).

## Patentansprüche

1. Verfahren zur Herstellung von Olefinen aus Erdgas (Naturgas), das Methan und Ethan enthält, dadurch gekennzeichnet, daß man
   a) das Erdgas (2) in zwei Fraktionen auftrennt, in eine an Methan angereicherte erste Gasfraktion (3) und in eine an Ethan angereicherte zweite Gasfraktion (4),
   b) die erste Gasfraktion mit molekularem Sauerstoff (6) bei (5) oxidiert in Gegenwart einer Kontaktmasse (Katalysatormasse), welche die oxidierende Kupplung des Methans zu höheren Kohlenwasserstoffen erlaubt,
   c) den Abstrom der Stufe (b) mit der an Ethan angereicherten Gasfraktion (7) der Stufe (a) mischt, wenn mindestens 80 % des molekularen Sauerstoffs in der Stufe (b) verbraucht worden sind, und
   d) die resultierende Mischung der Stufe (c) bei (8) pyrolysiert.

2. Verfahren nach Anspruch 1, in dem man in der Stufe (b) die an Methan angereicherte Fraktion (3) der Stufe (a) mit Wasserdampf mischt.

3. Verfahren nach einem der Ansprüche 1 und 2, in dem man in der Stufe (c) die an Ethan angereicherte Fraktion (4) der Stufe (a) mit einem oder mehr gesättigten Kohlenwasserstoffen, die nicht aus Erdgas stammen, mischt.

4. Verfahren nach einem der Ansprüche 1 und 2, in dem man in der Stufe (c) die an Ethan angereicherte Gasfraktion der Stufe (a) mit einem oder mehr Nicht-Kohlenwasserstoff-Gasen mischt.

5. Verfahren nach einem der Ansprüche 1 bis 4, in dem man die Stufen (b), (c) und (d) in einem Reaktor mit zirkulierendem fluidisiertem Bett (Wirbelbett) in Gegenwawrt einer Kontaktmasse durchführt.

6. Verfahren nach einem der Ansprüche 1 bis 4, in dem man die Stufen (b), (c) und (d) in einem Reaktor mit sprudelndem (siedendem) Bett in Gegenwart einer Kontaktmasse durchführt.

7. Verfahren nach einem der Ansprüche 1 bis 4, in dem man die Stufe (b) in einem Reaktor mit Fixbett in Gegenwart einer Kontaktmasse durchführt.

8. Verfahren nach einem der Ansprüche 1 bis 4, in dem man die Stufe (b) und mindestens eine der Stufen (c) und (d) in einem Reaktor mit Fixbett in Gegenwart einer Kontaktmasse durchführt.

9. Verfahren nach einem der Ansprüche 7 und 8, in dem die Kontaktmasse in dem Fixbett auf einen Monolithen als Träger aufgebracht ist.

10. Verfahren nach einem der Ansprüche 7 und 8, in dem die Kontaktmasse in dem Fixbett in Form eines Monolithen vorliegt.

11. Verfahren nach einem der Ansprüche 5 bis 10, in dem die Kontaktmasse der Formel entspricht

$$T_aLn_2O_{b-c}(CO_3)_c$$

worin T mindestens ein Erdalkalimetall und Ln mindestens ein Metall der Seltenen Erden bedeuten, a = 0,001 bis 1000, b = 3 + a, c = 0,1 bis b.

12. Verfahren nach einem der Ansprüche 5 bis 10, in dem die Kontaktmasse der Formel entspricht

$$T_aM_dLn_2O_{e-f}(CO_3)_f$$

worin T mindestens ein Erdalkalimetall, M mindestens ein Metall der Gruppe IVA des PSE und Ln mindestens ein Metall der Seltenen Erden bedeuten, a = 0,001 bis 1000, d = 0,001 bis 2, e = 3 + a + 2d-z, f = 0,1 bis e, z = 0 bis 0,5d.

13. Verfahren nach einem der Ansprüche 5 bis 10, bei dem die Kontaktmasse das Ergebnis der Imprägnierung von Magnesiumoxid mit einer wäßrigen Lösung von Lanthannitrat und Barium- oder Strontiumnitrat ist.

14. Verfahren nach einem der Ansprüche 1 bis 13, in dem man den Abstrom der Stufe (d) fraktioniert, dann die CO enthaltende Fraktion durch $H_2$ in $CH_4$ und $H_2O$ umwandelt und schließlich den resultierenden gasförmigen Abstrom in die Stufe (a) einführt.

## Claims

1. Process for producing olefins from natural gas containing methane and ethane, comprising :
   a) separating the natural gas (2) into two fractions, a first methane-enriched gas fraction (3) and a second ethane-enriched gas fraction (4),
   b) oxidizing (5) the first gas fraction with molecular oxygen (6), in the presence of a contact mass allowing the oxidizing coupling of the methane into superior hydrocarbons.
   c) mixing the effluent from stage (b) with the ethane-enriched gas fraction (7) from stage (a), when at least about 80 % by volume of the molecular oxygen has been already consumed in stage (b), and
   d) pyrolyzing (8) the mixture resulting from stage (c).

2. A process according to claim 1 wherein, in stage (b), the methane-enriched gas fraction (3) from stage (a) is mixed with water steam.

3. A process according to one of claims 1 and 2 wherein, in stage (c), the ethane-enriched gas fraction (4) from stage (a) is mixed with one or several saturated hydrocarbons which do not come from the natural gas.

4. A process according to one of claims 1 and 2 wherein, in stage (c), the ethane-enriched gas fraction (4) from stage (a) is mixed with one or several non hydrocarbon gases.

5. A process according to one of claims 1 to 4 wherein stages (b), (c) and (d) are performed in a circulating fluidized bed reactor in the presence of a contact mass.

6. A process according to one of claims 1 to 4 wherein stages (b), (c) and (d) are performed in a boiling bed reactor in the presence of a contact mass.

7. A process according to one of claims 1 to 4 wherein stage (b) is performed in a fixed bed reactor in the presence of a contact mass.

8. A process according to one of claims 1 to 4 wherein stages (b), and at least one of stages (c) and (d) are performed in a fixed bed reactor in the presence of a contact mass.

9. A process according to one of claims 7 and 8 wherein the contact mass in fixed bed is supported on a monolith.

10. A process according to one of claims 7 and 8 wherein the contact mass in fixed bed is in the form of a monolith.

11. A process according to one of claims 5 to 10 in which the contact mass corresponds to the formula $T_aLn_2O_{b-c}(CO_3)_c$ where T represents at least one alkaline-earth metal, Ln represents at least one rare earth metal, a = 0.001 to 1.000, b = 3 + a, c = 0.1 to 10.

12. A process according to one of the claims 5 to 10 in which the contact mass corresponds to the formula $T_aM_dLn_2O_{e-f}(CO_3)_f$, where T represents at least one alkaline-earth metal, M represents at least one metal from group IVA, $L_n$, represents at least one rare earth metal, a = 0.001 to 1.000, d = 0.001 to 2, e = 3 + a+d-z, f=0.1 to e, z=O to 0.5d.

13. A process according to one of the claims 5 to 10 in which the contact mass results from the impregnation of magnesia with an aqueous solution of lanthanum nitrate and barium and/or strontium nitrate.

14. A process according to one of the claims 1 to 13 in which the effluent from stage (d) is fractionated, then the CO containing fraction is converted to $CH_4$ and $H_2O$ by $H_2$ and finally the resulting gaseous effluent is sent back to stage (a).